**Europäisches Patentamt**

**European Patent Office**

**Office européen des brevets**

(19)

(11) Publication number : **0 318 560 B1**

# EUROPEAN PATENT SPECIFICATION

(12)

(45) Date of publication of patent specification :
**29.01.92 Bulletin 92/05**

(51) Int. Cl.⁵ : **A61K 31/475,** A61K 31/19,
// A61K47/00, A61K9/08

(21) Application number : **88905413.6**

(22) Date of filing : **10.06.88**

(86) International application number :
**PCT/HU88/00042**

(87) International publication number :
**WO 88/09662 15.12.88 Gazette 88/27**

(54) **ANTIPSORIATIC PHARMACEUTICAL COMPOSITION AND PROCESS FOR THE PREPARATION.**

(30) Priority : **12.06.87 HU 267987**

(43) Date of publication of application :
**07.06.89 Bulletin 89/23**

(45) Publication of the grant of the patent :
**29.01.92 Bulletin 92/05**

(84) Designated Contracting States :
**BE CH DE FR GB IT LI NL**

(73) Proprietor : **RICHTER GEDEON VEGYESZETI GYAR R.T.**
**Gyömröi ut 19-21**
**H-1445 Budapest (HU)**

(72) Inventor : **KEVE, Tibor**
**Hunyadlejto " 28**
**H-1121 Budapest (HU)**
Inventor : **SZPORNY, Lászl**
**Szabolcska M. u. 7**
**H-1114 Budapest (HU)**
Inventor : **KIRALY, Arpád**
**Mályva u. 17**
**H-1141 Budapest (HU)**
Inventor : **FEKETE, György**
**Széher u. 62**
**H-1021 Budapest (HU)**
Inventor : **FORG CS, Lilla**
**Galamb u. 7**
**H-1052 Budapest (HU)**

Inventor : **STEFKO, Béla**
**Orlay u. 2/b**
**H-1117 Budapest (HU)**
Inventor : **ZSADON, Béla**
**Villányi t 64/b**
**H-1113 Budapest (HU)**
Inventor : **GALAMBOS, János**
**Cserkut u. 40**
**H-1162 Budapest (HU)**
Inventor : **BALAZSNE ZAYER, Mária**
**Zalka M. tér 1**
**H-1110 Budapest (HU)**
Inventor : **Hangay, György**
**Rajk Lászl u. 35-37**
**H-1136 Budapest (HU)**
Inventor : **KELEN, András**
**Somfa köz 6**
**H-1107 Budapest (HU)**
Inventor : **BUKOVECZNE BARTA, Margit**
**Kisfaludy u. 40**
**H-1082 Budapest (HU)**
Inventor : **KARPATI, Egon**
**Mihályfi E. u. 7/b**
**H-1022 Budapest (HU)**
Inventor : **KISS, Béla**
**Vöröshadsereg tja 197/c**
**H-2220 Vecsés (HU)**
Inventor : **S S, Gyöngyvér**
**Mályva u. 17**
**H-1141 Budapest (HU)**

(74) Representative : **Tubby, David George et al**
**MARKS & CLERK 57-60 Lincoln's Inn Fields**
**London WC2A 3LS (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 318 560 B1

## Description

The invention relates to an antipsoriatic pharmaceutical composition containing 17,18-dehydroapovincaminol-3′,4′,5′-trimethoxybenzoate or pharmaceutically acceptable acid addition salts thereof and to a process for preparing it.

It is known from the literature [Ann. Rev. Pharmacol. Toxicol. 17, 441 (1977)] that in the case of several diseases such as asthma, psoriasis, or thrombosis, a pathologically high level of the phosphodiesterase enzyme can be observed in the affected tissues. It has also been proved for the psoriasis [Arch. Dermatol. 104, 352 (1971)] that substances inhibiting the action of phosphodiesterase are potentially useful for the treatment of this illness.

Based on this novel approach differing from the traditional methods of the psoriasis therapy, the preparation and successful use of the phosphodiesterase-inhibiting 17,18-dehydroapovincaminol-3′,4′,5′-trimethoxybenzoate and its acid addition salts in the treatment of psoriasis were disclosed in the Hungarian patent specification No. 183,323 (equivalent to the Belgian patent specification No. 892,069), according to which the active ingredient was used in a formulation containing it partly in a heterogeneous phase, partly in a dissolved form.

The various toxicological and preclinical investigations carried out on this formulation in the meantime gave newer results.

In addition to the therapeutic effect observed, a dose-dependance was searched for formulations containing the active ingredient in a heterogeneous phase. It has been proved in our experiments that, in the above formulations, a part of the active ingredient only contributes to the action whereby a whole bioavailability cannot be achieved.

Based on the description cited above, the dose-dependance can be stated as a consequence of the limited solubility for formulations containing the active ingredient in a dissolved form; however, there is no possibility to develop an optimum concentration of the formulation. Thus, the development and the therapeutical use of an optimal pharmaceutical formulation are made difficult by these problems arising from the physical properties of the active ingredient.

Thus, the aim of the invention is to provide an antipsoriatic pharmaceutical composition and a process for incorporating the active ingredient into the pharmaceutical formulation in a dissolved form, with essentially higher concentration limits than earlier.

It is known from the literature that, on using analogous compounds with an eburnane skeleton as medicaments, the active ingredient can be brought into solution, if desired, in various pharmaceutically acceptable organic solvents (alcohols, esters) in the presence of water and a solubilizing agent (polyvinylpyrrolidone, tensides) or in a mixture thereof. However, it has been unsuccessful till now to adjust the desired concentration interval of 17,18-dehydroapovincaminol-3′,4′,5′-trimethoxybenzoate or its pharmaceutically acceptable acid addition salts by using these methods and simultaneously, in the course of the formulating, the active ingredient was precipitated from the solution by the eventually required dilution with water.

It has been recognized in the course of our experiments that, on preparing formulations containing 17,18-dehydroapovincaminol-3′,4′,5′-trimethoxybenzoate or its pharmaceutically acceptable acid addition salts as active ingredient, the active ingredient could be dissolved in the presence of a higher than stoichiometric amount of pharmaceutically acceptable organic acids in water, alcohols or aqueous alcohols as pharmaceutically acceptable carriers within significantly wider concentration limits than by using the methods described earlier. Thus, the whole bioavailability of the active ingredient can be utilized, the optimal therapeutical dose can be established and simultaneously, an improved resorption of the active ingredient can be promoted by using a solubilizing acid.

In the case of several formulations such as ointments, creams or gels, a further advantage resides in the surprising recognition that a stable gel is formed without any additive by the solution prepared according to the invention in the course of its dilution with water within defined limits if a defined ratio of the active ingredient to the solubilizing acid is used. It can be stated that the active ingredient is present in a homogeneous, molecular distribution in this gel.

Thus, in a first aspect, the present invention provides a homogeneous pharmaceutical composition comprising a therapeutically acceptable solution of 17,18-dehydroapovincaminol-3′,4′,5′-trimethoxybenzoate, or a pharmaceutically acceptable acid addition salt thereof, as active ingredient, with a stoichiometric excess of a therapeutically acceptable organic acid having no basic group, the acid being soluble in water, monohydric alcohols, polyhydric alcohols and/or mixtures thereof, the composition preferably containing 1. 1-50 equivalents, especially 5-20 equivalents, of acid to active ingredient.

In another aspect, the present invention also provides a process for the preparation of a composition as described above, comprising dissolving one equivalent of active ingredient in 1. 1 to 50, preferably 5 to 20, equivalents of a therapeutically acceptable organic acid containing no basic group and being soluble in water or in a water-miscible monovalent or polyvalent alcohol or with a mixture thereof, to form a therapeutically acceptable solution, and

a) formulating the solution as a pharmaceutical composition _per se_ or, if desired, together with therapeutically active agents showing no synergism with the active ingredient and/or with pharmaceutical auxiliary materials (additives),

or

b) diluting the solution with 5 to 50 parts by weight of water and formulating the thus-obtained gel, which contains the active ingredient in a homogeneous molecular distribution, _per se_ or together with therapeutically active agents showing no synergism with the active ingredient and/or with pharmaceutically acceptable auxilliary materials to form a pharmaceutical composition.

In the process of the invention, the solvent is the solubilizing therapeutically acceptable acid or optionally a solution thereof in water or in water-miscible monovalent or polyvalent alcohols or in their mixtures. If the acids are liquid (such as lactic or acetic acic), the active ingredient may be dissolved in the acid itself.

If the acid is solid (such as tartaric or ascorbic acid), the active ingredient is dissolved in an ethanolic or aqueous ethanolic solution of the acid.

Ethanol, propylene glycol or glycerol may preferably be used as monovalent or polyvalent alcohols.

As solubilizing agents therapeutically acceptable organic acids may be used which do not contain any basic group and are soluble in water or in water-miscible monovalent or polyvalent alcohols or in their mixtures. In the case of acids containing a basic group, the active ingredient cannot be dissolved in the above system or the solution or gel, respectively, obtained is unstable whereby the active ingredient precipitates from the solution.

The solubilizing acid is used in an amount of 1.1 to 50, preferably 5 to 20, equivalents as calculated for the active ingredient. If the molar excess is low (e.g. 1.1 to 5 equivalents), a lower concentration maximum will be achieved on dissolution, or the gel prepared will not be hard enough from the viewpoint of formulating. On increasing the molar excess (up to 5 to 20 equivalents) both the obtainable concentration maximum and the hardness of the gel prepared become higher.

If the amount of the solubilizing acid is between 20 and 50 equivalents, the irritating effect of the composition also increases with the increasing concentration and gel hardness; over 50 equivalents, the composition cannot be used therapeutically because of the irritating effect.

The resorption-increasing action of the solubilizing acid was verified by a study carried out with the tritiated active ingredient.

In the course of the resorption study made with the tritiated active ingredient, a suspension, an acid-free solution and an acid-solubilized solution of the same concentration (2 %) were compared on rats with an external application. Based on these experiments, the resorption showed the following order:

Acid-solubilized solution > solubilized solution> > suspension.

The higher therapeutical bioavailability of the active ingredient was verified in clinical trials carried out on patients suffering from psoriasis. In this study, a suspension ointment containing 2 % of active ingredient was compared to an acid-solubilized ointment containing 0.2 % of active ingredient.

On using the acid-solubilized ointment containing 0.2 % of active ingredient, the same recovery ratio was observed as compared to the suspension composition containing 2 % of the active ingredient. Within this comparison study, 50 % of the patients gave an advantageous response to the treatment; 25 % showed a good reaction; and 25 % did not give any response. It seems unambiguously from these data that one tenth of the active ingredient (0.2 % of acid-solubilized active ingredient) resulted in the same preferable clinical experiences as observed on the treatment with the suspension ointment containing 2 % of active ingredient.

The process of the invention is illustrated in detail by the following non-limiting Examples.

**Example 1**

17,18-Dehydroapovincaminol-3′,4′,5′-trimethoxybenzoate in a water-in-oil type ointment

Composition:                                                    g

17,18-Dehydroapovincaminol-3',4',5'-tri-

methoxybenzoate                                                 2.0

Lactic acid                                                     3.0

5-Chloro-2-(2,4-dichlorophenoxy)-phenol

(triclosane)                                                    0.1

Magnesium sulfate                                               0.5

Aerosil R-972 ®                                                 1.0

White wax (cera alba)                                           1.5

Elfacos ST 37 (emulsifier; manufacturer:

AKZO, The Netherlands)                                          1.5


Composition:                                                    g

Propylene glycol                                                3.0

Cetiol V (emulsifier; manufacturer:

Henkel, FRG)                                                    5.0

Neo PCL Selbstemulgierend (emulsifier;

Dragoco, Austria)                                               23.0

Distilled water                                                 59.4


The active ingredient is dissolved in lactic acid, propylene glycol is added to the solution and after adding an aqueous solution of triclosane and magnesium sulfate, a gel is prepared. A melt of the remaining components kept at 50 °C is added to the gel heated to 50 °C and the thus-obtained cream is stirred until it cools down.

**Example 2**

17,18-Dehydroapovincaminol-3',4',5'-trimethoxybenzoate in a tincture

4

| Composition: | % |
|---|---|
| 17,18-Dehydroapovincaminol-3',4',5'-tri-methoxybenzoate | 2.0 |
| Triamcinolone acetonide | 0.1 |
| Tartaric acid | 3.0 |
| Propylene glycol | 30.0 |
| Ethanol 96 % | up to 100.0 |

A tincture is prepared by using the above components (ingredients).

**Example 3**

The process of Example 1 is followed, except that 2.0 g of 17,18-dehydroapovincaminol-3',4',5'-trimethoxybenzoate hydrochloride are used as active ingredient.

**Example 4**

The process of Example 2 is followed, except that 15.0 % of glycerol are used instead of propylene glycol.

**Claims**

1. A homogeneous pharmaceutical composition comprising a therapeutically acceptable solution of 17,18-dehydroapovincaminol-3',4',5'-trimethoxybenzoate, or a pharmaceutically acceptable acid addition salt thereof, as active ingredient, with a stoichiometric excess of a therapeutically acceptable organic acid having no basic group, the acid being soluble in water, monohydric alcohols, polyhydric alcohols and/or mixtures thereof.

2. A composition according to Claim 1 containing 1. 1-50 equivalents, especially 5-20 equivalents, of acid to active ingredient.

3. A composition according to claim 1 or 2 comprising the hydrochloride salt as the active ingredient.

4. A composition according to any preceding claim comprising a solvent as defined, especially ethanol, polyethyleneglycol, 1,2-propanediol and/or glycerol.

5. A composition according to any preceding claim in the form of a cream or a gel and comprising 5-50 parts by weight of water.

6. A composition according to any preceding claim formulated with pharmaceutically auxiliary materials.

7. A composition according to any preceding claim wherein the acid is selected from lactic and tartaric acids.

8. A process for the preparation of a composition according to any preceding claim comprising dissolving one equivalent of active ingredient in 1. 1 to 50, preferably 5 to 20, equivalents of a therapeutically acceptable organic acid containing no basic group and being soluble in water or in a water-miscible monovalent or polyvalent alcohol or with a mixture thereof, to form a therapeutically acceptable solution, and

   a) formulating the solution as a pharmaceutical composition per se or, if desired, together with therapeutically active agents showing no synergism with the active ingredient and/or with pharmaceutical auxiliary materials (additives),

   or

   b) diluting the solution with 5 to 50 parts by weight of water and formulating the thus-obtained gel, which contains the active ingredient in a homogeneous molecular distribution, per se or together with therapeutically active agents showing no synergism with the active ingredient and/or with pharmaceutically acceptable auxiliary materials to form a pharmaceutical composition.

EP 0 318 560 B1

**Patentansprüche**

1. Homogene pharmazeutische Zusammensetzung, umfassend eine therapeutisch geeignete Lösung von 17,18-Dehydroapovincaminol-3',4',5'-trimethoxybenzoat oder von dessen pharmazeutisch geeignetem Säureadditionssalz als aktiven Bestandteil mit einem stöchiometrischen Überschuss einer therapeutisch geeigneten organischen Säure, die keine basischen Gruppen hat, wobei die Säure in Wasser, einwertigen Alkoholen, mehrwertigen Alkoholen und/oder Mischungen davon löslich ist.

2. Zusammensetzung nach Anspruch 1, die 1,1 bis 50 Äquivalente, insbesondere 5 bis 20 Äquivalente Säure zu dem aktiven Bestandteil hat.

3. Zusammensetzung nach Anspruch 1 oder 2, die das Hydrochloridsalz als aktiven Bestandteil enthält.

4. Zusammensetzung nach einem der vorangegangenen Ansprüche, die ein Lösungsmittel, wie definiert, insbesondere Ethanol, polyethylenglykol, 1,2-Propandiol und/oder Glyzerin enthält.

5. Zusammensetzung nach einem der vorangegangenen Ansprüche in Form einer Creme oder eines Gels, die 5 bis 50 Gew.-Teile Wasser enthält.

6. Zusammensetzung nach einem der vorangegangenen Ansprüche formuliert mit pharmazeutischen Hilfsmaterialien.

7. Zusammensetzung nach einem der vorangegangenen Ansprüche, bei der die Säure aus Milchsäure und Weinsäure ausgewählt ist.

8. Verfahren zur Herstellung einer Zusammensetzung nach einem der vorangegangenen Ansprüche, umfassend das Lösen eines Äquivalents von aktivem Bestandteil in 1,1 bis 50, vorzugsweise 5 bis 20 Äquivalenten einer therapeutisch geeigneten, organischen Säure, die keine basischen Gruppen enthält, und in Wasser oder in einem mit Wasser mischbaren einwertigen oder mehrwertigen Alkohol oder einer Mischung davon löslich ist, wobei eine therapeutisch geeignete Lösung gebildet wird, und

(a) Formulieren der Lösung als pharmazeutische Zusammensetzung per se oder, falls gewünscht, zusammen mit therapeutisch aktiven Bestandteilen, die keinen Synergismus mit dem aktiven Bestandteil zeigen, und/oder mit pharmazeutischen Hilfsmaterialien (Additiven) oder
(b) Verdünnen der Lösung mit 5 bis 50 Gew.-Teilen Wasser und Formulieren des so erhaltenen Gels, das den aktiven Bestandteil in homogener molekularer Verteilung enthält, per se oder zusammen mit therapeutisch aktiven Bestandteilen, die keinen Synergismus mit dem aktiven Bestandteil zeigen, und/oder mit pharmazeutisch geeigneten Hilfsmaterialien, wobei eine pharmazeutische Zusammensetzung gebildet wird.

**Revendications**

1. Composition pharmaceutique homogène comprenant une solution acceptable du point de vue thérapeutique de 3',4',5'-triméthoxybenzoate de 17, 18-déhydroapovincominol ou d'un sel d'addition d'acide de celui-ci acceptable du point de vue pharmaceutique en tant que composant actif, avec un excès stoechiométrique d'un acide organique acceptable du point de vue thérapeutique n'ayant aucun groupe basique, l'acide étant soluble dans l'eau, des alcools monohydriques, des alcools polyhydriques et/ou leurs mélanges.

2. Composition suivant la revendication 1 contenant 1,1 à 50 équivalents, en particulier 5 à 20 équivalents d'acide par rapport au composant actif.

3. Composition suivant la revendication 1 ou la revendication 2 comprenant le chlorhydrate en tant que composant actif.

4. Composition suivant l'une quelconque des revendications précédentes comprenant un solvant tel que défini, en particulier l'éthanol, un polyéthylène glycol, le 1,2-propanediol et/ou le glycérol.

5. Composition suivant l'une quelconque des revendications précédentes sous la forme d'une crème ou d'un gel et comprenant 5 à 50 parties en poids d'eau.

6. Composition suivant l'une quelconque des revendications précédentes formulée avec des matières auxiliaires du point de vue pharmaceutique.

7. Composition suivant l'une quelconque des revendications précédentes dans laquelle l'acide est choisi parmi l'acide lactique et l'acide tartrique.

8. Procédé pour la préparation d'une composition suivant l'une quelconque des revendications précédentes, comprenant la dissolution d'un équivalent de composant actif dans 1,1 à 50, de préférence 5 à 20 équivalents d'un acide organique acceptable du point de vue pharmaceutique ne contenant aucun groupe basique et étant soluble dans l'eau ou dans un alcool monovalent ou polyvalent miscible à l'eau ou un mélange de ceux-ci, pour former une solution acceptable du point de vue thérapeutique, et

a) la formulation de la solution sous forme de composition pharmaceutique per se ou, si cela est désiré,

6

avec des agents actifs du point de vue thérapeutique ne présentant aucune synergie avec le composant actif et/ou avec les matières auxiliaires du point de vue pharmaceutique (additifs),

ou

b) la dilution de la solution avec 5 à 50 parties en poids d'eau et la formulation du gel ainsi obtenu, qui contient le composant actif dans une distribution moléculaire homogène, per se ou avec des agents actifs du point de vue thérapeutique ne présentant aucune synergie avec le composant actif et/ou avec les matières auxiliaires du point de vue pharmaceutique pour former une composition pharmaceutique.